# EUROPEAN PATENT APPLICATION

(11) **EP 4 790 147 A1**
(43) Date of publication of application: **12.08.2026**
(21) Application number: 24874036.7
(22) Date of filing: 28.08.2024
(51) Int. Cl.: C12Q 1/6886, C12N 15/12, C12Q 1/6869, C12N 15/11

(54) **NOVEL DNA METHYLATION MARKER TAGME-6 AND USE THEREOF AS TARGET FOR TUMOR DIAGNOSIS**

(30) Priority: 05.10.2023 CN 202311288769
(71) Applicant: Shanghai Epiprobe Biotechnology Co., Ltd., Shanghai 200233 (CN)
(72) Inventor: LI, Zhenyan, Shanghai 200233 (CN); TANG, Jialin, Shanghai 200233 (CN); LI, Wei, Shanghai 200233 (CN)
(74) Representative: Oryon NV
(86) International application number: PCT/CN2024/115172
(87) International publication number: WO 2025/073217

(57) **Abstract**

Provided are a novel DNA methylation marker TAGMe-6 and a use thereof as a target for tumor diagnosis. The abnormal hypermethylation of a specific site in the TAGMe-6 can be used as a basis for tumor diagnosis, and is suitable for various tumors, including solid tumors and non-solid tumors. The TAGMe-6 can be used as a marker for multiple types of tumors, and is used for early screening, molecular diagnosis, and prognosis or curative effect assessment of clinical tumors.

## Description

This application claims priority to the patent application with application number CN 202311288769.3, filed on October 5, 2023; the entire contents of which are incorporated herein by reference.

### TECHNICAL FIELD

The present disclosure belongs to the field of epigenetics; more specifically, the present disclosure relates to a novel DNA methylation marker TAGMe-6 and use thereof as target for tumor diagnosis.

### BACKGROUND OF THE DISCLOSURE

Malignant tumors are diseases that pose a serious threat to human health. They are neoplasms formed by the clonal abnormal proliferation of a cell in a local tissue under the action of various carcinogenic factors, resulting from the loss of normal growth regulation at the genetic level. The etiology of tumors is extremely complex, and prevention of most tumors at the etiological level is difficult. The occurrence and development of tumors are gradual processes, and most patients are already in the middle or advanced stage at the time of diagnosis, losing the opportunity for radical treatment. Traditional methods for treating tumors comprise surgical treatment, radiotherapy, and chemotherapy. However, these traditional treatment methods all have limitations, and there is often a lack of suitable treatment options for many patients with mid-to-advanced stage tumors. The development of safe and efficient biotherapeutic approaches has become a focus of attention in the field of tumor therapy. Gene therapy refers to a treatment that introduces an exogenous functional gene into target cells to correct congenital metabolic disorders, compensate for gene deficiencies or provide new functions, with the aim of inhibiting and killing tumor cells. Constructing efficient and safe gene delivery vectors is key to tumor gene therapy, which also necessitates early intervention. Therefore, early detection, diagnosis and treatment are key to improving the cure rate and treatment prognosis.

Tumor markers refer to substances that exist in malignant tumor cells, substances that are abnormally produced by malignant tumor cells, or substances that are produced by the host in response to tumor stimulation, reflecting tumor occurrence and development and monitoring the response to treatment. Tumor markers are present in tissues, body fluids, and excreta of tumor patients and can be detected by immunological, biological, and chemical methods. Common tumor markers comprise hormones, enzymes, proteins, etc., comprising carcinoembryonic antigen (CEA), alpha-fetoprotein (AFP), neuron-specific enolase (NSE) and carbohydrate antigen 125 (CA125). Such markers have been applied in clinical practice, but their sensitivity and specificity cannot meet practical needs.

During the process of normal cells transforming into tumor cells, a type of chemical modification on DNA, DNA methylation, undergoes dynamic changes with tumor progression. Numerous studies have confirmed that DNA methylation plays a role in regulating the expression of oncogenes and tumor suppressor genes. Compared to genetic mutations, DNA methylation can be detected at an early stage of cellular transformation. DNA methylation is relatively stable and can be detected from minimal sample material, providing feasibility for its clinical application.

Therefore, finding molecular targets that can be used for the diagnosis, prognosis, and prediction of cancer occurrence and development is of great significance for early cancer screening, clinical intervention, and guiding patient treatment.

### SUMMARY OF DISCLOSURE

The object of the disclosure is to provide a novel DNA methylation marker TAGMe-6 and use thereof as target for tumor diagnosis.

In the first aspect, the present disclosure provides a use of an isolated polynucleotide or a polynucleotide converted therefrom in the preparation of an agent or a kit for detecting tumors, wherein the polynucleotide comprises: (1) a polynucleotide TAGMe-6 with a nucleotide sequence as shown in SEQ ID NO: 1, or a polynucleotide fragment comprising at least one CpG site with modification thereof; or (2) a polynucleotide complementary in sequence to the polynucleotide or fragment of (1); wherein the polynucleotide converted from the isolated polynucleotide is a polynucleotide corresponding to (1) or (2), wherein unmodified cytosine is converted to T or U, while cytosine C of the CpG site(s) with modification is unchanged.

As an optional embodiment, the modification comprises 5-methylation modification (5mC), 5-hydroxymethylation modification (5hmC), 5-formylcytosine modification (5-fC), or 5-carboxylcytosine modification (5-caC).

As an optional embodiment, the detection comprises diagnosis, detection, screening or prognostic evaluation.

As an optional embodiment, SEQ ID NO: 1 further comprises a sequence variant thereof, or a homologous sequence thereof.

As an optional embodiment, the sequence variant or homologous sequence is a sequence having at least 80%, at least 85%, at least 90%, at least 92%, at least 95%, at least 96%, at least 98%, at least 99%, at least 99.5%, or at least 99.8% sequence identity to the sequence shown in SEQ ID NO: 1. Correspondingly, it also comprises polynucleotides converted from the sequence variant or homologous sequence (wherein unmodified cytosine is converted to T or U, while cytosine C of the CpG site(s) with modification is unchanged).

As an optional embodiment, the polynucleotide converted from the isolated polynucleotide is a polynucleotide with a nucleotide sequence as shown in SEQ ID NO:2.

As an optional embodiment, the at least one CpG site with modification is any "CpG" selected from sites Nos. 1 to 152 (such as sites Nos. 2 to 152, more specifically such as sites Nos. 3, 5, 10, 14, 15, 20, 25, 30, 35, 40, 50, 60, 70, 80, 100, 120, 140, 150) of the polynucleotide with the nucleotide sequence as shown in SEQ ID NO: 1, or a combination thereof; preferably any CpG site selected from sites Nos. 55 to 68, sites Nos. 1 to 54, or sites Nos. 69 to 152 of the polynucleotide with the nucleotide sequence as shown in SEQ ID NO: 1, or a combination thereof.

As an optional embodiment, the polynucleotide fragment is a polynucleotide with a nucleotide sequence shown in positions 619 to 703 or positions 589 to 731 of SEQ ID NO: 1.

As an optional embodiment, the tumors comprise (but are not limited to): respiratory system tumors, digestive system tumors, urinary system tumors, gynecological and reproductive system tumors, hematological system tumors, nervous system tumors, head and neck tumors, skin system tumors, endocrine system tumors or skeletal system tumors.As an optional embodiment, the tumors comprise: lung cancer, liver cancer, prostate cancer, cervical cancer, endometrial cancer, urothelial carcinoma, biliary tract tumor, gastric carcinoma, breast cancer, esophageal carcinoma, brain glioma, colorectal cancer, leukaemia, pancreatic cancer, thyroid cancer, melanoma, nasopharyngeal carcinoma, oral carcinoma, throat cancer, osteosarcoma, lymphadenoma, renal cell carcinoma or ovarian cancer.

As an optional embodiment, the detection is directed to a tumor (comprising early-stage, middlestage or late-stage tumors) or a precancerous lesion thereof.

As an optional embodiment, samples for detecting tumors comprise: tissue samples, body fluid samples, blood samples.

As an optional embodiment, the samples comprise (but are not limited to): paraffin-embedded samples, pleural effusion samples, alveolar lavage fluid samples, ascites and lavage fluid samples, bile samples, stool samples, urine samples, saliva samples, sputum samples, cerebrospinal fluid samples, cell smear samples, cervical scraping or brushing samples, tissue and cell biopsy samples, and so on.

In another aspect, the present disclosure provides a method for preparing an agent for detecting tumors, comprising: (a) providing an isolated polynucleotide or a polynucleotide converted therefrom, comprising (1) a polynucleotide TAGMe-6 with a nucleotide sequence as shown in SEQ ID NO: 1, or a polynucleotide fragment comprising at least one CpG site with modification thereof; or (2) a polynucleotide complementary in sequence to the polynucleotide or fragment of (1); wherein the polynucleotide converted from the isolated polynucleotide is a polynucleotide corresponding to (1) or (2), wherein unmodified cytosine is converted to T or U, while cytosine C of the CpG site(s) with modification is unchanged; preferably, the at least one CpG site with modification is any CpG site selected from sites Nos. 1 to 152 of the polynucleotide with the nucleotide sequence as shown in SEQ ID NO: 1, or a combination thereof; preferably any CpG site selected from sites Nos. 55 to 68, sites Nos. 1 to 54, or sites Nos. 69 to 152 of the polynucleotide with the nucleotide sequence as shown in SEQ ID NO: 1, or a combination thereof; preferably, the polynucleotide fragment is a polynucleotide with a nucleotide sequence shown in positions 619 to 703 or positions 589 to 731 of SEQ ID NO: 1; (b) using the polynucleotide of (a) as a target sequence, designing a detection agent that specifically detects the modification on CPG site(s) of the target sequence.

As an optional embodiment, the agent for detecting tumors comprises, but is not limited to: a primer, a probe, a chip or a test strip.

As an optional embodiment, one or more sets of agents can be prepared for the target sequence.

As an optional embodiment, the detection agent is integrated onto a chip.

In another aspect, the present disclosure provides an agent or a combination of agents for specifically detecting the modification on CPG site(s) of a target sequence, wherein the target sequence is: (1) a polynucleotide TAGMe-6 with a nucleotide sequence as shown in SEQ ID NO: 1, or a polynucleotide fragment comprising at least one CpG site with modification thereof; or (2) a polynucleotide complementary in sequence to the polynucleotide or fragment of (1); wherein the polynucleotide converted from the isolated polynucleotide is a polynucleotide corresponding to (1) or (2), wherein unmodified cytosine is converted to T or U, while cytosine C of the CpG site(s) with modification is unchanged; preferably, the agent or the combination of agents is for a gene sequence comprising the target sequence, preferably, the gene sequence comprises gene Panels or gene groups; preferably, the agent or the combination of agents comprises: primers for amplifying the region from positions 619 to 703 or from positions 589 to 731 of the nucleotide sequence shown in SEQ ID NO: 1.

As an optional embodiment, the agent or the combination of agents is: primers comprising the sequence at positions 29 to 58 of SEQ ID NO: 3 and the sequence at positions 28 to 55 of SEQ ID NO: 4.

As an optional embodiment, the agent or the combination of agents is: primers having the sequences of SEQ ID NO: 3 and SEQ ID NO: 4.

As an optional embodiment, the agent or the combination of agents is: primers formed by linking any one of the sequences shown in SEQ ID NOs: 7-16 to the sequence at positions 29 to 58 of SEQ ID NO: 3, and primers formed by linking any one of the sequences shown in SEQ ID NOs: 17-26 to the sequence at positions 28 to 55 of SEQ ID NO: 4.

As an optional embodiment, the agent or the combination of agents is: primers having the sequences shown in SEQ ID NO: 27 and SEQ ID NO: 28.

As an optional embodiment, the agent or the combination of agents is: primers having the sequences shown in SEQ ID NO: 29 and SEQ ID NO: 30.

In another embodiment, the agent or the combination of agents also comprises: primers having the sequences shown in SEQ ID NO: 5 and SEQ ID NO: 6.

In another aspect, the present disclosure provides a use of the agent or the combination of agents for preparing kits for detecting tumors.

In another aspect, the present disclosure provides a kit for detecting tumors, comprising the agent or the combination of agents.

As an optional embodiment, the kit may further comprise, but is not limited to: a DNA purification reagent, a DNA extraction reagent, bisulfite, a PCR amplification reagent.

As an optional embodiment, the kit further comprises: an instruction for indicating the detection operation steps and result judgment criteria.

In another aspect, the present disclosure provides a method for analyzing the methylation level of a test sample, comprising: (i) extracting a polynucleotide from a test sample; and (ii) analyzing the modification on CPG site(s) of a target sequence or a fragment thereof within the extracted polynucleotide, wherein the target sequence is: (1) a polynucleotide TAGMe-6 with a nucleotide sequence as shown in SEQ ID NO: 1, or a polynucleotide fragment comprising at least one CpG site with modification thereof; or (2) a polynucleotide complementary in sequence to the polynucleotide or fragment of (1); wherein the polynucleotide converted from the isolated polynucleotide is a polynucleotide corresponding to (1) or (2), wherein unmodified cytosine is converted to T or U, while cytosine C of the CpG site(s) with modification is unchanged.

As an optional embodiment, the method for detecting the modification on CPG site(s) of a target sequence within the extracted polynucleotide comprises: pyrosequencing, bisulfite conversion sequencing, methylation-specific PCR, methylation-sensitive restriction enzyme digestion, method using methylation chip, qPCR, digital PCR, next-generation sequencing, third-generation sequencing, whole-genome methylation sequencing, DNA enrichment detection, simplified bisulfite sequencing, HPLC, MassArray, or a combination thereof.

As an optional embodiment, the method for analyzing the modification on CPG site(s) of a target sequence within the extracted polynucleotide comprises: (i) treating the extracted polynucleotide to convert unmodified cytosines therein to uracil; preferably, the modification comprises 5-methylation modification, 5-hydroxymethylation modification, 5-formylcytosine modification or 5-carboxylcytosine modification; preferably, treating the nucleic acid of step (i) with bisulfite; and (ii) analyzing the modification of the target sequence in the nucleic acid treated by (i).

As an optional embodiment, treating the nucleic acid of step (i) with bisulfite; and (ii) analyzing the modification of the target sequence in the nucleic acid treated by (i).

As an optional embodiment, an abnormal methylation level refers to hypermethylation of the C in the CpG of the polynucleotide.

As an optional embodiment, other methylation detection methods and newly developed methylation detection methods in the future can also be applied to the present disclosure.

As an optional embodiment, the method for analyzing the methylation level is not a diagnostic method, i.e., it is not for the purpose of directly obtaining a diagnosis result of a disease.

As an optional embodiment, the method for detecting the methylation level of a sample is an in vitro method.

As an optional embodiment, the methylation-sensitive restriction enzyme is a restriction enzyme that is sensitive to the presence of a methylated base within its recognition site; comprising but not limited to: HhaI, BmgBI, HaeII, RruI, TaiI, Bsu15I, Hin6I, HpyCH4IV, NarI, or a combination of one or more thereof.

In another aspect, the present disclosure provides an isolated polynucleotide or a polynucleotide converted therefrom, wherein the polynucleotide comprises: (1) a polynucleotide TAGMe-6 with a nucleotide sequence as shown in SEQ ID NO: 1, or a polynucleotide fragment comprising at least one CpG site with modification thereof; preferably, the at least one CpG site with modification is any CpG site selected from sites Nos. 55 to 68, sites Nos. 1 to 54, or sites Nos. 69 to 152 of the polynucleotide with the nucleotide sequence as shown in SEQ ID NO: 1, or a combination thereof; preferably the polynucleotide fragment is a nucleotide with the nucleotide sequence shown in positions 619 to 703 or positions 589 to 731 of SEQ ID NO: 1; (2) a polynucleotide complementary in sequence to the polynucleotide or fragment of (1); wherein the polynucleotide converted from the isolated polynucleotide is a polynucleotide corresponding to (1) or (2), wherein unmodified cytosine is converted to T or U, while cytosine C of the CpG site(s) with modification is unchanged.

Other aspects of the disclosure will be apparent to those skilled in the art based on the disclosure herein.

### BRIEF DESCRIPTION OF DRAWINGS

Figure 1. Methylation level (left panel), specificity and sensitivity (right panel) of TAGMe-6 in lung cancer tissues and control tissues.
Figure 2. Methylation level (left panel), specificity and sensitivity (right panel) of TAGMe-6 in liver cancer tissues and control tissues.
Figure 3. Methylation level (left panel), specificity and sensitivity (right panel) of TAGMe-6 in prostate cancer tissues and control tissues.
Figure 4. Methylation level (left panel), specificity and sensitivity (right panel) of TAGMe-6 in cervical cancer tissues and control tissues.
Figure 5. Methylation level (left panel), specificity and sensitivity (right panel) of TAGMe-6 in endometrial cancer tissues and control tissues.
Figure 6. Methylation level (left panel), specificity and sensitivity (right panel) of TAGMe-6 in urothelial carcinoma tissues and control tissues.
Figure 7. Methylation level (left panel), specificity and sensitivity (right panel) of TAGMe-6 in biliary tract tumor tissues and control tissues.
Figure 8. Methylation level (left panel), specificity and sensitivity (right panel) of TAGMe-6 in gastric carcinoma tissues and control tissues.
Figure 9. Methylation level (left panel), specificity and sensitivity (right panel) of TAGMe-6 in breast cancer tissues and control tissues.
Figure 10. Methylation level (left panel), specificity and sensitivity (right panel) of TAGMe-6 in esophageal carcinoma tissues and control tissues.
Figure 11. Methylation level (left panel), specificity and sensitivity (right panel) of TAGMe-6 in brain glioma tissues and control tissues.
Figure 12. Methylation level (left panel), specificity and sensitivity (right panel) of TAGMe-6 in colorectal cancer tissues and control tissues.
Figure 13. Methylation level (left panel), specificity and sensitivity (right panel) of TAGMe-6 in leukaemia tissues and control tissues.
Figure 14. Methylation level (left panel), specificity and sensitivity (right panel) of TAGMe-6 in pancreatic cancer tissues and control tissues.
Figure 15. Methylation level (left panel), specificity and sensitivity (right panel) of TAGMe-6 in thyroid cancer tissues and control tissues.
Figure 16. Methylation level (left panel), specificity and sensitivity (right panel) of TAGMe-6 in melanoma tissues and control tissues.
Figure 17. Methylation level (left panel), specificity and sensitivity (right panel) of TAGMe-6 in nasopharyngeal carcinoma tissues and control tissues.
Figure 18. Methylation level (left panel), specificity and sensitivity (right panel) of TAGMe-6 in oral carcinoma tissues and control tissues.
Figure 19. Methylation level (left panel), specificity and sensitivity (right panel) of TAGMe-6 in throat cancer tissues and control tissues.
Figure 20. Methylation level (left panel), specificity and sensitivity (right panel) of TAGMe-6 in osteosarcoma tissues and control tissues.
Figure 21. Methylation level (left panel), specificity and sensitivity (right panel) of TAGMe-6 in lymphadenoma tissues and control tissues.
Figure 22. Methylation level (left panel), specificity and sensitivity (right panel) of TAGMe-6 in renal cell carcinoma tissues and control tissues.
Figure 23. Methylation level (left panel), specificity and sensitivity (right panel) of TAGMe-6 in ovarian cancer tissues and control tissues.
Figure 24. BSP assay for methylation levels of methylation (CpG) sites Nos. 1 to 54 of SEQ ID NO: 1 in cancer cells and normal control cells.
Figure 25. BSP assay for methylation levels of methylation sites Nos. 69 to 152 of SEQ ID NO: 1 in cancer cells and normal control cells.

### DETAILED DESCRIPTION

Based on extensive research and screening, the present disclosure reveals a novel tumor marker related to epigenetic modification, named as TAGMe-6. The phenomenon of abnormally high methylation at specific sites in TAGMe-6 can be used as a basis for tumor diagnosis, and it is applicable to various types of tumors, comprising solid tumors and non-solid tumors. It can serve as a pan-cancer marker for early screening, molecular diagnosis, prognosis or efficacy assessment of clinical tumors.

In the present disclosure, the term "tumor" refers to a broad spectrum of tumors (pan-cancer), wherein the SEQ ID NO: 1 region in the genome exhibits a hypermethylated state as described in the present disclosure. It may be a solid tumor or a non-solid tumor, and may comprise (but is not limited to): respiratory system tumors, digestive system tumors, urinary system tumors, gynecological and reproductive system tumors, hematological system tumors, nervous system tumors, head and neck tumors, skin system tumors, endocrine system tumors or skeletal system tumors.

As used herein, "sample" comprises substances suitable for DNA extraction and DNA methylation detection obtained from any individual or isolated tissue, cell or body fluid (such as plasma). For example, the samples comprise but are not limited to: tissue samples, paraffin embedded samples, blood samples, pleural effusion samples and alveolar lavage fluid samples, ascites and lavage fluid samples, bile samples, stool samples, urine samples, saliva samples, cerebrospinal fluid samples, cell smear samples, cervical scraping or brushing samples, tissue and cell biopsy samples.

As used herein, "hypermethylation" refers to high level of methylation, hydroxymethylation, formylcytosine or carboxylcytosine of CpG in a gene sequence. For example, in the case of methylation specific PCR (MSP), if the PCR reaction with methylation specific primers has positive PCR results, the DNA (gene) region of interest is in hypermethylation state. For another example, in the case of real-time quantitative methylation specific PCR, hypermethylation can be determined based on statistic difference of the methylation status value as compared with the control sample.

In the present disclosure, the methylation status of the nucleotide sequence shown in SEQ ID NO: 1 or a partial region (fragment) thereof is significantly different between tumor tissues and non-tumor tissues. When an abnormally high methylation state is detected in the gene sequence region, the subject can be considered to have a tumor or be at high risk for tumors. This significant difference in the methylation status exhibited by the gene sequence shown in SEQ ID NO: 1 or a partial region thereof is very prominently present in a variety of tumors (comprising early-stage tumors).

The present disclosure also comprises "conservative variation sequences" of the sequence shown in SEQ ID NO: 1 (or the reverse complementary sequence thereof) that are conservative or have high sequence identity to the nucleotide sequence shown in SEQ ID NO: 1 (or the reverse complementary sequence thereof). The term "high sequence identity" refers to, for example, higher than 90%, higher than 92%, higher than 95%, higher than 98%, higher than 99%, etc. It should be understood that individual sequence variations (e.g., some meaningless single nucleotide polymorphisms (SNPs)) may exist among different biological individuals, but this does not affect the detection based on the overall scheme of the present disclosure.

Based on the above, the present disclosure provides a nucleic acid derived from a specific region of the human genome, wherein it has the gene sequence shown in SEQ ID NO:1 or a partial region thereof, and also comprises the antisense chain thereof. In tumor cells, 5-methylcytosine (5mC) or other similar epigenetic modifications are generated at the C position of multiple 5'-CpG-3' bases in the nucleic acid sequence.

Detection of one or more CpG sites provided by the present disclosure is possible. Therefore, the present disclosure also comprises fragments of the nucleic acid of the nucleotide sequence, comprising at least one methylated CpG site. The at least one CpG site can comprise 1 to 152 CpG sites, more specifically such as 2, 3, 5, 8, 10, 12, 15, 20, 25, 30, 35, 40, 50, 60, 70, 80, 100, 120, 140, 150 CpG sites of the polynucleotide with the nucleotide sequence as shown in SEQ ID NO: 1, or a reverse complementary sequence thereof. Those skilled in the art can understand that, after the numbering of CpG sites based on one DNA strand is provided in the present disclosure, the numbering of each CpG site in the complementary DNA strand corresponding to the sense strand is readily obtainable based on the content provided by the present disclosure.

Based on the information of the specific fragment in the human genome provided by the present disclosure, those skilled in the art can readily obtain the CpG site and apply it. The embodiments of the present disclosure provide a series of sequence fragments comprising CpG sites. These fragments can serve as examples of preferred embodiments, but it should be understood that based on the information provided by the present disclosure, variations can be made, such as selecting a longer sequence comprising the sequence of the present disclosure, or selecting a sequence that overlaps regionally with the relative sequence of the present disclosure.

Gene Panels or gene groups comprising the nucleotide sequence shown in SEQ ID NO: 1, a sequence fragment thereof, or a complementary sequence thereof are also encompassed by the disclosure. For the gene panel or gene group, the characteristics of normal cells and tumor cells can also be identified through DNA methylation detection.

It should be understood that a wide variety of methods that can be used to analyze methylation status can be applied to the present disclosure, and the present disclosure is not particularly limited to such detection methods. The nucleic acids provided by the present disclosure can serve as key regions for analyzing methylation status in the genome. Their methylation status can be analyzed by various methods known in the art, thereby analyzing the occurrence or development of tumors.

After treatment with bisulfite, un-methylated cytosines in the nucleic acid described in SEQ ID NO: 1 of the present disclosure, or a fragment or complementary sequence thereof, will be converted to uracil, while methylated cytosines remain unchanged. Therefore, the present disclosure also provides nucleic acids obtained by treating the above-mentioned nucleic acids (comprising the complementary chain (antisense chain)) with bisulfite, comprising: the nucleic acid or nucleic acid fragment with the nucleotide sequence as shown in SEQ ID NO: 2. These nucleic acids can serve as more direct targets for designing detection agents or detection kits.

The nucleic acid having the nucleotide sequence shown in SEQ ID NO: 1 of the present disclosure and/or a complementary nucleic acid thereof and/or one or more fragments thereof can be integrated into one or several sets, such as one or several nucleic acid sets, for use by those skilled in the art, e.g., selecting one or more nucleic acids or nucleic acid fragments from the set to design targeted detection agents. The designed targeted detection agents can also be integrated into one or several sets, such as one or several kits.

The nucleic acids converted from the nucleic acid having the nucleotide sequence shown in SEQ ID NO: 1 of the present disclosure and/or a complementary nucleic acid thereof and/or one or more fragments thereof (e.g., after being treated with bisulfite) can also be integrated into one or several sets, such as one or several nucleic acid sets, for use by those skilled in the art, e.g., selecting one or more nucleic acids or nucleic acid fragments from the set to design targeted detection agents. The designed targeted detection agents can also be integrated into one or several sets, such as one or several kits, or one or several chips.

Based on the disclosure of the target gene and the epigenetic characteristics thereof, these methods well-known in the art, as well as some methods to be developed, can be applied to the present disclosure to carry out the detection of methylation level. Determining the methylation level of a nucleic acid can be performed by existing methods (such as methylation-specific PCR (MSP) or real-time quantitative methylation-specific PCR, Methylight), or other methods still under development or to be developed in the future. For example, quantitative methylation-specific PCR (QMSP) can be used to detect methylation levels. It is based on continuous optical monitoring of fluorescent PCR and is more sensitive than MSP. It has high throughput and avoids analyzing results by electrophoresis. In addition, other available methods comprise: qPCR (Me-qPCR), next-generation sequencing, pyrosequencing, Sanger sequencing, bisulfite conversion sequencing, whole-genome methylation sequencing, DNA enrichment detection, reduced representation bisulfite sequencing, HPLC, combined gene group detection methods and other conventional methods in the art. Although some preferable examples are provided in the embodiments of the present disclosure, the overall scheme of the present disclosure is not limited thereto.

As a preferable embodiment of the present disclosure, a method for detecting the methylation profile of a nucleic acid in a sample in vitro is also provided. The method is based on the principle that bisulfite can convert un-methylated cytosine to uracil, which is then converted to thymine during subsequent PCR amplification, while methylated cytosine remains unchanged; thus, after treating the nucleic acid with bisulfite, methylated sites generate a C/T-like nucleic acid polymorphism (SNP). Based on the above principle, identifying the methylation profile of a nucleic acid in a test sample can effectively distinguish between methylated and un-methylated cytosines.

The method of the present disclosure comprises: first, providing a sample and extracting genomic DNA; second, treating the genomic DNA of step (a) with bisulfite, thereby converting un-methylated cytosines in the genomic DNA to uracil; third, analyzing whether there is an abnormal methylation profile in the genomic DNA treated in step (b).

The method of the present disclosure can be used for: testing a subject's sample to assess whether the subject has a tumor; or for distinguishing populations at high risk for tumors. The method may be for purposes not aimed at directly obtaining a disease diagnosis result, such as situations not aimed at determining the final disease outcome, regional population analysis studies, scientific research, population census, etc.

In a preferable embodiment of the present disclosure, DNA methylation is detected by PCR amplification and pyrosequencing. Practical applications are not limited to this method; other DNA methylation detection methods known in the art or currently being improved can also be used. In PCR amplification, the primers used are not limited to those provided in the embodiments; primers that differ in sequence from those provided in the embodiments of the present disclosure but still target the nucleic acids or corresponding CpG sites indicated by the present disclosure can also be obtained.

As a preferable embodiment of the present disclosure, a method for detecting the methylation status of a nucleic acid in a sample in vitro is also provided, which is the methylation-sensitive restriction endonuclease (MSRE) method. When a methylated base is present in its cleavage site, the methylation-sensitive restriction endonuclease cannot cleave the DNA. The MSRE method is based on the fundamental principle that methylation-sensitive type II restriction endonucleases cannot cleave fragments comprising one or more methylated cleavage site sequences. Fragments comprising one or more methylated CpG sequences are cleaved with methyl-sensitive type II endonucleases and their isoschizomers (insensitive to methylation), and then analyzed by Southern blotting. Advantages of the method comprise: no need for detailed information on the primary structure of the target DNA, and it can provide a direct assessment of CpG island methylation status, comprising obtaining some quantitative analysis information on the methylation of the tested gene.

Other detection methods and agents known to those skilled in the art for determining genome sequences, their variations, and methylation status can be comprised in the present disclosure, centering on the marker nucleic acid provided by the present disclosure.

The present disclosure provides a method for preparing a tumor detection agent, comprising: providing the nucleic acid, using the full length or a fragment of the nucleic acid as a target sequence, and designing a detection agent that specifically detects the target sequence; wherein the target sequence comprises at least one methylated CpG site. The detection agent may comprise, but is not limited to: a chip, a primer, a probe, etc.; after obtaining the marker, selection of the detection agent is within the ability of those skilled in the art.

After knowing the sequence of the nucleic acid, designing primers is known to those skilled in the art. The two primers are located on either side of the specific sequence of the target gene to be amplified (comprising the CpG sequence; complementarity to CpG targets the originally methylated gene region, while complementarity to CpG targets the originally unmethylated gene region). In a preferable embodiment of the present disclosure, the agent is a primer, and the primers are preferably those listed in the embodiments. Besides primers, other diagnostic or detection agents can also be prepared, comprising but not limited to probes, chips, etc.

The agent can also be an agent combination, such as a primer combination. For example, the combination comprises more than one set of primers, so that the multiple nucleic acids mentioned above can be amplified separately.

The present disclosure also provides a kit for detecting the methylation profile of a nucleic acid in a sample in vitro, comprising: a container, and the above-mentioned primer pair located in the container.

The kit may also comprise various reagents required for DNA extraction, DNA purification, PCR amplification, etc., and other reagents, such as sample processing reagents. In addition, the kit may also comprise an instruction for use, indicating the detection operation steps and result judgment criteria, to facilitate application by those skilled in the art.

The methods and agents of the present disclosure have very high accuracy when used for clinical tumor diagnosis, as reflected in the detection of clinical samples of various tumors in the embodiments of the present disclosure. The present disclosure can be applied in fields such as early tumor screening, efficacy determination, auxiliary diagnosis, prognosis monitoring, etc., or in situations not aimed at directly obtaining a disease diagnosis result as mentioned above.

The pan-cancer marker provided by the present disclosure can be applied to detection using cervical exfoliated cells, etc. The test sample is easily and non-invasively obtained. Compared to the clinical requirement of surgically obtaining tissue samples, this method significantly reduces patient discomfort and improves compliance.

The disclosure is further illustrated by the drawings and examples described below. These examples are merely illustrative for the embodiments of the present disclosure, and do not limit the scope of the present disclosure.

### Example 1. Determination of the Methylation Detection Target

1. Acquisition of the human TAGMe-6 gene sequence SEQ ID NO: 1;

GGCTCCTGCAGCTCCCGCCGAAGACTCAAAGCCAGAGTGGACATAAACGCCGTGGGCAGGACCCCGGTGAGGCCTCGGTGCCC
TTCTTGGGGGTTCTCAGCGTTGGCCCAGAAGCCTCAGCCCGGGTCTAAGAACTTGGGACTCTCCTCGACTTTGGCGATCGGCC
GGGTCATCCGGCTCAGGGCCTCAGCGGCAGCGGGCAAAACTCTAGTAGGAGTCTCTTGCCGAGGGCGTGTTTCGACGTCAGAG
CCAAACTCGGGACAGACTAGCCAAGCGCGGACGGCGCGAGAGTGGCTGGCAGCGCCAGCACGCAGCCTGGGTTCAGAGCAAGG
CTGGGCGCTCTCAGCAAAGGGCGGCCTGGGGCTGCGCGGGCGGCGGACTGCAGGCGGGAGAAGAGCGAGGTGCGCCAGGCTC
TGGGGCGCGCAACTGCCCAGCCTCGTGAAAGATCGCGCCGCAGATGGGGCGCAGCTGCGCGCTCACTCGTGTGGACTGGAAA
CGCTCCGAGCCGGTTATTTTAAAAACCGGGAAATAAGGCGGGTTCCCTCTTCGCCCGCCACTTCCCACCAAGTAGGCTGTGCG
GCCCTGGGGGCTGACTGTCCTCAAGCAGCCAGGCTCCACCGCGCGCCGCGCTGCGCCGAGGTCCGCTCTGCCGCAGGGACGC
TGGCAGCCCGTTGAACACCGGCAAGAGCGCCAGAGGCTAGCGGCCGCCAGGATCTCTACCAGGCTCTGCTCGCACCCGCCTG
CCTCCCTTTCGTTTGGCCTGTCCTCCGTTCAACTGAAATCGTTAATTTTCTTACCCCCTTGTTCTCATTTTGATATATTCTAC
GCTTTAAACATGCTCCGTTTTCTTTTGTTTAGTCTGCTCCCTCCCTCTTTGTCCTTTCCCCCTTCTCTAGTTATCCGTTTCGT
TCGATCTTGCTCCTGCTTTTTTTATTCGTTCGTTCCTCATTTATTCATTTTAGTTCATCCCAGCTCGCCGACTGCCATTTACC
CTCTCGTTCTCGCCGCGCTCTCCGTTGTTTTGTTCAATTTCCCTTCCCCTTTTCTTGGTTGTCGCTCGCTTTCTTTGGTTTTC
TTTCTCGGTATTTCGTTGTCAAGGCCACCCTTGCCGTCGGATCCCGGGGTGCTGGGTTTCTCCCGGCCGCTCGTTCCGCACC
AGCGCTCTCTGCAGTTCGCGCGGCACCGGTGTGGTCCGGGGGCCCGAGCTGTCGGTGCCGGATGCGGCGCGCCTAGCAGGGA
CGCGGGCCTGGGGGGGTGGCTCCTGCCCGACGCGGAGCGCTGAGCCAGGCCGGGTACCTGTCTCTGGCGGTGCTCACCGCAC
TGCGCGGCCTCTGCCGTCTGGCTGGGATCAGAGGAGCCAGGCCAACTGCTTCTCATTAAGTCCCAACTGTGGTTTTTATCAGG
AAAGCCTCTTTCAAAGGGCACAGACACGAAGCTCCGCGGACTCGTTCATTTCCTCCGTTGACCCACACACACCTCCCCGCCCT
CCCCTACACATTCCCACCGCCCCGGCTGGGCGAAAGCCGGAGATGCCCGGCCACTCCGTGGAGGCCCGCGAGGCGCCAGCCG
GGCGGCGGCAGGGGGTTGAGGCGGATCTTGGAGGATCCAGTTCTGGGCCTAGGCTGCGGGATATGGCAGCGCAGATAAGGTGG
GTGCAGTGCGGAAGCCGAGACGCCTTACAGGTCATAGGGTGCGGCGGACGGCCGCAGAGCTGCCGATCAGCCTGCCAGG
(SEQ ID NO: 1)

In the above sequence (sense strand), each "CG" indicated by a solid underline represents a methylated CpG site, sequentially numbered from 5' to 3' as CpG sites Nos. 1 to 152 (the 1st to 152nd methylated CpG sites). The regions marked by dashed underlines correspond to regions designed for upstream primers and downstream primers in certain embodiments; the bold italic regions correspond to target regions for detection in certain embodiments.

### 1.2 Acquisition of the bisulfite-converted DNA sequence SEQ ID NO: 2, wherein Y represents C or U (T):

GGTTTTGTAGTTTTYGTYGAAGATTTAAAGTTAGAGTGGATATAAAYGTYGTGGGTAGGATTTYGGTGAGGTTTYGGTGTTT
TTTTTGGGGGTTTTTAGYGTTGGTTTAGAAGTTTTAGTTYGGGTTTAAGAATTTGGGATTTTTTTYGATTTTGGYGATYGGTY
GGGTTATTYGGTTTAGGGTTTTAGYGGTAGYGGGTAAAATTTTAGTAGGAGTTTTTTGTYGAGGGYGTGTTTYGAYGTTAGAG
TTAAATTYGGGATAGATTAGTTAAGYGYGGAYGGYGYGAGAGTGGTTGGTAGYGTTAGTAYGTAGTTTGGGTTTAGAGTAAGG
TTGGGYGTTTTTAGTAAAGGGYGGTTTGGGGTTGYGYGGGYGGYGGATTGTAGGYGGGAGAAGAGYGAGGTGYGTTAGGTTTT
GGGGYGYGTAATTGTTTAGTTTYGTGAAAGATYGYGTYGTAGATGGGGYGTAGTTGYGYGTTTATTYGTGTGGATTGGAAAY
GTTTYGAGTYGGTTATTTTAAAAATYGGGAAATAAGGYGGGTTTTTTTTTYGTTYGTTATTTTTTATTAAGTAGGTTGTGYGG
TTTTGGGGGTTGATTGTTTTTAAGTAGTTAGGTTTTATYGYGYGTYGYGTTGYGTYGAGGTTYGTTTTGTYGTAGGGAYGTT
GGTAGTTYGTTGAATATYGGTAAGAGYGTTAGAGGTTAGYGGTYGTTAGGATTTTTATTAGGTTTTGTTYGTATTYGTTTGT
TTTTTTTTYGTTTGGTTTGTTTTTYGTTTAATTGAAATYGTTAATTTTTTTATTTTTTTGTTTTTATTTTGATATATTTTAYG
TTTTAAATATGTTTYGTTTTTTTTTGTTTAGTTTGTTTTTTTTTTTTTTGTTTTTTTTTTTTTTTTTAGTTATTYGTTTYGTT
YGATTTTGTTTTTGTTTTTTTTATTYGTTYGTTTTTTATTTATTTATTTTAGTTTATTTTAGTTYGTYGATTGTTATTTATTTT
TTYGTTTTYGTYGYGTTTTTYGTTGTTTTGTTTAATTTTTTTTTTTTTTTTTTTGGTTGTYGTTYGTTTTTTTTGGTTTTTTTT
TTYGGTATTTYGTTGTTAAGGTTATTTTTGTYGTYGGATTTYGGGGTGTTGGGTTTTTTTYGGTYGTTYGTTTYGTATTAGYGT
TTTTTGTAGTTYGYGYGGTATYGGTGTGGTTYGGGGGTTYGAGTTGTYGGTGTYGGATGYGGYGYGTTTAGTAGGGAYGYGGGT
TTGGGGGGGTGGTTTTTGTTYGAYGYGGAGYGTTGAGTTAGGTYGGGTATTTGTTTTTGGYGGTGTTTATYGTATTGYGYGGTT
TTTGTYGTTTGGTTGGGATTAGAGGAGTTAGGTTAATTGTTTTTTATTAAGTTTTAATTGTGGTTTTTATTAGGAAAGTTTTTT
TTAAAGGGTATAGATAYGAAGTTTYGYGGATTYGTTTATTTTTTTYGTTGATTTATATATATTTTTTYGTTTTTTTTTATATAT
TTTTATYGTTTYGGTTGGGYGAAAGTYGGAGATGTTYGGTTATTTYGTGGAGGTTYGYGAGGYGTTAGTYGGGYGGYGGTAGGG
GGTTGAGGYGGATTTTGGAGGATTTAGTTTTGGGTTTAGGTTGYGGGATATGGTAGYGTAGATAAGGTGGGTGTAGTGYGGAAG
TYGAGAYGTTTTATAGGTTATAGGGTGYGGYGGAYGGTYGTAGAGTTGTYGATTAGTTTGTTAGG (SEQ ID NO: 2)

In the above sequence (sense strand), each "YG" indicated by a solid underline represents a converted methylated CpG site, sequentially numbered from 5' to 3' as "YG" Nos. 1 to 152 (the 1st to 152nd converted methylated CpG sites). The regions marked by dashed underlines correspond to regions designed for upstream primers and downstream primers in certain embodiments; the bold italic regions correspond to target regions for detection in certain embodiments.

3. The region for detection is determined, with primers designed at the upstream and downstream regions of the region.

### Example 2. Design and synthesis of detection agents

2.1 First-round PCR primers with a length of 25-35 bp and moderate CG content were designed and the amplification length was ensured to be 100-300 bp.

2.2 Barcodes (Sample-ID) were added to the ends of the first-round primers.

2.3 Tag sequences were added to the ends of the first-round primers for library construction.

2.4 The first-round PCR primers and the second-round PCR primers with Illumina adapter and indexes were synthesized.

**Table 1**

| Prime r Name | 5'-3' Sequence | Notes |
|---|---|---|
| F1 | tacacgacgctcttccgatctCGTGATYGTTGATTGTTTT TAAGTAGTTAGGTTTTAT (SEQ ID NO: 3) | First-round PCR forward primer; wherein positions 29-58 are bases complementary to the sequence of target (upstream and downstream) region for detection, positions 22-28 are the sequence of tag and positions 1-21 are Illumina adapter. |
| R1 | gacgtgtgctcttccgatctTAGCTTRAACAAAACCTAA TAAAAATCCTAACRAC (SEQ ID NO: 4) | First-round PCR reverse primer; wherein positions 28-55 are bases complementary to the sequence of target (upstream and downstream) region for detection, positions 21-27 are the sequence of tag and positions 1-20 are Illumina adapter. |
| F2 | AATGATACGGCGACCACCGAGATCTACACTCT TTCCCTACACGACGCTCTTCCGATCT (SEQ ID NO: 5) | Second-round PCR forward primer |
| R2 | CAAGCAGAAGACGGCATACGAGATCGTGATG TGACTGGAGTTCAGACGTGTGCTCTTCCGATC T (SEQ ID NO: 6) | Second-round PCR reverse primer |

F1 and R1 amplify the sequence region corresponding to positions 589-731 (comprising CpG sites Nos. 55-69) in SEQ ID NO: 1 or SEQ ID NO: 2, wherein the region comprising CpG sites Nos. 55-68 is positions 619-703.

### Example 3. Verification of primers

A pair of primers was synthesized to perform two rounds of PCR on positive and negative reference samples:
The first-round PCR reaction system was shown in Table 2.

**Table 2**

| PCR-1 | System | PCR Conditions | | |
|---|---|---|---|---|
| 2x taq mix | 5 µL | 98°C | 30s | 1 cycle |
| F/R primer (0.5uM) | 3 µL | 98 °C | 10s | 30 cycles |
| Template | 2 µL | 58°C | 30s | |
| Total | 10 µL | 72°C | 30s | |
| | | 72°C | 3min | 1 cycle |
| | | 8°C | - | - |

The second-round PCR reaction system was shown in Table 3.

**Table 3**

| PCR-2 | System | PCR Conditions | | |
|---|---|---|---|---|
| 2x taq mix | 9µL | 98°C | 30s | 1 cycle |
| F/R primer (1uM) | 2µL | 98°C | 10s | 30 cycle |
| Template (First-round product) | 2µL | 62°C | 30s | |
| ddH2O | 7µL | 72°C | 30s | |
| Total | 20 µL | 72°C | 3min | 1 cycle |
| | | 8°C | - | - |

### Example 4. Construction of Primer Pool

After the PCR efficiency of the primers was verified, a primer pool with different barcodes was synthesized, and primers were diluted and combined. The first-round PCR F primers and R primers have M×N (10×10) combinations, allowing simultaneous detection and localization of multiple samples. In Table 4, the lowercase bases in the F1 primers correspond to positions 1-21 of the forward primer F1 in Table 1 (Illumina adaptor), the uppercase bases are sequencing tag, and the sequence following the tag is connected to bases complementary to the sequence of target region for detection (connected to positions 29-58 of SEQ ID NO: 3 in subsequent examples). The lowercase bases in the R1 primers correspond to positions 1-20 of the reverse primer R1 in Table 1, the uppercase bases are sequencing tag, and the sequence following the tag is connected to bases complementary to the sequence of target region for detection (connected to positions 28-55 of SEQ ID NO: 4 in subsequent examples). The second-round primers are F2 and R2 from Table 1.

**Table 4**

| F1 | 5'-3' Primer Sequence | R1 | 5'-3' Primer Sequence |
|---|---|---|---|
| F1-1 | tacacgacgctcttccgatctCG TGATY (SEQ ID NO: 7) | R1-1 | gacgtgtgctcttccgatctTAGCTTR (SEQ ID NO: 17) |
| F 1-2 | tacacgacgctcttccgatctACATCGY (SEQ ID NO: 8) | R1-2 | gacgtgtgctcttccgatctGG CTA CR (SEQ ID NO: 18) |
| F1-3 | tacacgacgctcttccgatctGCCTAAY (SEQ ID NO: 9) | R1-3 | gacgtgtgctcttccgatctCTTGTAR (SEQ ID NO: 19) |
| F1-4 | tacacgacgctcttccgatctTGG TCAY (SEQ ID NO: 10) | R1-4 | gacgtgtgctcttccgatctAG TCAAR (SEQ ID NO: 20) |
| F 1-5 | tacacgacgctcttccgatctCACTGTY (SEQ ID NO: 11) | R1-5 | gacgtgtgetcttccgatctAG TTCC R (SEQ ID NO: 21) |
| F1-6 | tacacgacgctcttccgatctATTGGCY (SEQ ID NO: 12) | R1-6 | gacgtgtgctcttccgatctCCGACCR (SEQ ID NO: 22) |
| F 1-7 | tacacgacgctcttccgatctGATCTGY (SEQ ID NO: 13) | R1-7 | gacgtgtgctcttccgatctGGACGGR (SEQ ID NO: 23) |
| F1-8 | tacacgacgctcttccgatctTCAAGTY (SEQ ID NO: 14) | R1-8 | gacgtgtgctcttccgatctCTCTACR (SEQ ID NO: 24) |
| F 1-9 | tacacgacgetcttccgatctCTGATCY (SEQ ID NO: 15) | R1-9 | gacgtgtgctcttccgatctGCGGACR (SEQ ID NO: 25) |
| F1-10 | tacacgacgctcttccgatctAAGCTA Y (SEQ ID NO: 16) | R1-10 | gacgtgtgetetteegatetTG TGATR (SEQ ID NO: 26) |

In Table 4, R represents A or G.

### Example 5. Differential methylation of TAGMe-6 CpG sites in tumor tissues and non-tumor cells

NGS sequencing is used with the following steps:
1. Clinical samples were obtained: Paracancerous/non-cancer and cancer tissue samples were collected clinically. Paracancerous/non-cancer samples were used as the control group, and cancer tissue samples were used as the experimental group for tumor detection.
2. DNA was extracted: DNA was extracted from the experimental group and the control group, respectively. In this experiment, the adsorption column method was used for extraction (other methods are also applicable).
3. Bisulfite was used for treatment: The extracted DNA samples were treated with bisulfite, strictly following the procedure. In this experiment, the EZ DNA Methylation-Gold Kit (ZYMO Research, Cat# D5006) was used (other kits are also applicable).
4. Using the primers from the primer pool (first-round PCR primers) and the Illumina system universal sequencing primers (second-round PCR primers), two rounds of PCR amplification were performed by conventional methods to construct an NGS library.
5. After PCR amplification, the specificity of the PCR fragments was detected by 2% agarose gel electrophoresis. 5 µL of the PCR product from each sample was pooled, purified, and the target fragment library was recovered for NGS sequencing.
6. Sequencing results were analyzed: Sequencing information for each sample was extracted based on the primer barcode sequences.
7. TAGMe-6 methylation value was calculated: NGS sequencing can independently detect the methylation status of individual CpG sites within the target region. The median methylation value of all CpG sites was calculated as the methylation value of TAGMe-6 in the sample.
8. Results were analyzed: The methylation values of TAGMe-6 in non-tumor tissues and tumor tissues were compared, and the cutoff value was determined by ROC curve analysis.

### Example 6. TAGMe-6: Lung cancer clinical sample assay - NGS sequencing

20 paracancerous samples of lung cancer were obtained and used as the control group. 20 lung cancer samples were obtained and used as the experimental group. According to the primer combination described in Example 4 above, two rounds of PCR were performed to construct an NGS library of lung cancer clinical samples. The methylation level of TAGMe-6 was analyzed following the NGS sequencing procedure.

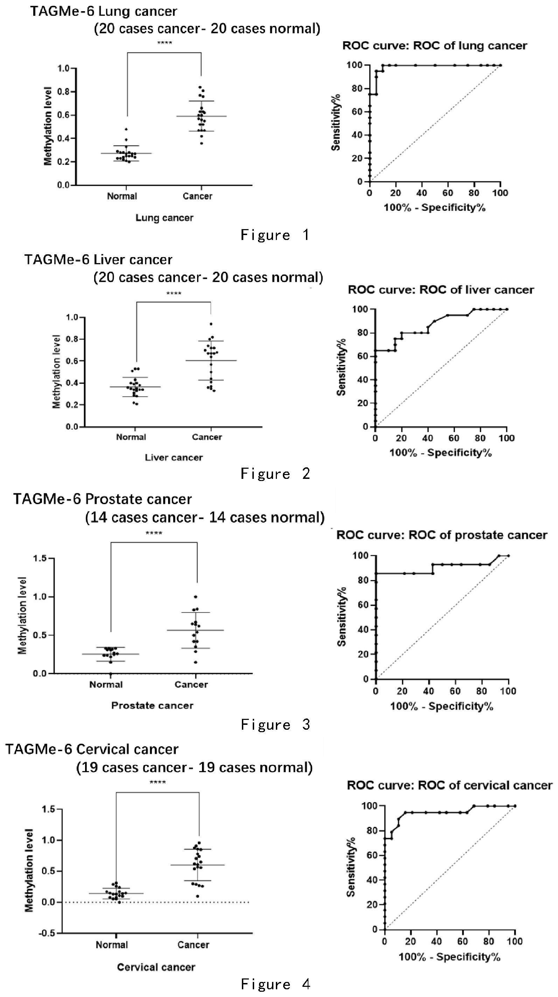

The results in Figure 1 demonstrated that in clinical samples of lung cancer, the methylation value of TAGMe-6 in cancer tissues was significantly higher than that in paracancerous tissues (****P < 0.0001). Sensitivity was 95% and specificity was 95%.

### Example 7. TAGMe-6: Liver cancer clinical sample assay - NGS sequencing

20 paracancerous samples of liver cancer were obtained and used as the control group. 20 liver cancer samples were obtained and used as the experimental group. According to the primer combination described in Example 4 above, two rounds of PCR were performed to construct an NGS library of liver cancer clinical samples. The methylation level of TAGMe-6 was analyzed following the NGS sequencing procedure.

The results in Figure 2 demonstrated that in clinical samples of liver cancer, the methylation value of TAGMe-6 in cancer tissues was significantly higher than that in paracancerous tissues (****P < 0.0001). Sensitivity was 80% and specificity was 80%.

### Example 8. TAGMe-6: Prostate cancer clinical sample assay - NGS sequencing

14 paracancerous samples of prostate cancer were obtained and used as the control group. 14 prostate cancer samples were obtained and used as the experimental group. According to the primer combination described in Example 4 above, two rounds of PCR were performed to construct an NGS library of prostate cancer clinical samples. The methylation level of TAGMe-6 was analyzed following the NGS sequencing procedure.

The results in Figure 3 demonstrated that in prostate cancer clinical samples, the methylation value of TAGMe-6 in cancer tissues was significantly higher than that in paracancerous tissues (****P < 0.0001). Sensitivity was 85.71% and specificity was 100%.

### Example 9. TAGMe-6: Cervical cancer clinical sample assay - NGS sequencing

19 paracancerous samples of cervical cancer were obtained and used as the control group. 19 cervical cancer samples were obtained and used as the experimental group. According to the primer combination described in Example 4 above, two rounds of PCR were performed to construct an NGS library of cervical cancer clinical samples. The methylation level of TAGMe-6 was analyzed following the NGS sequencing procedure.

The results in Figure 4 demonstrated that in cervical cancer clinical samples, the methylation value of TAGMe-6 in cancer tissues was significantly higher than that in paracancerous tissues (****P < 0.0001). Sensitivity was 89.47% and specificity was 89.47%.

### Example 10. TAGMe-6: Endometrial cancer clinical sample assay - NGS sequencing

19 paracancerous samples of endometrial cancer were obtained and used as the control group. 19 endometrial cancer samples were obtained and used as the experimental group. According to the primer combination described in Example 4 above, two rounds of PCR were performed to construct an NGS library of endometrial cancer clinical samples. The methylation level of TAGMe-6 was analyzed following the NGS sequencing procedure.

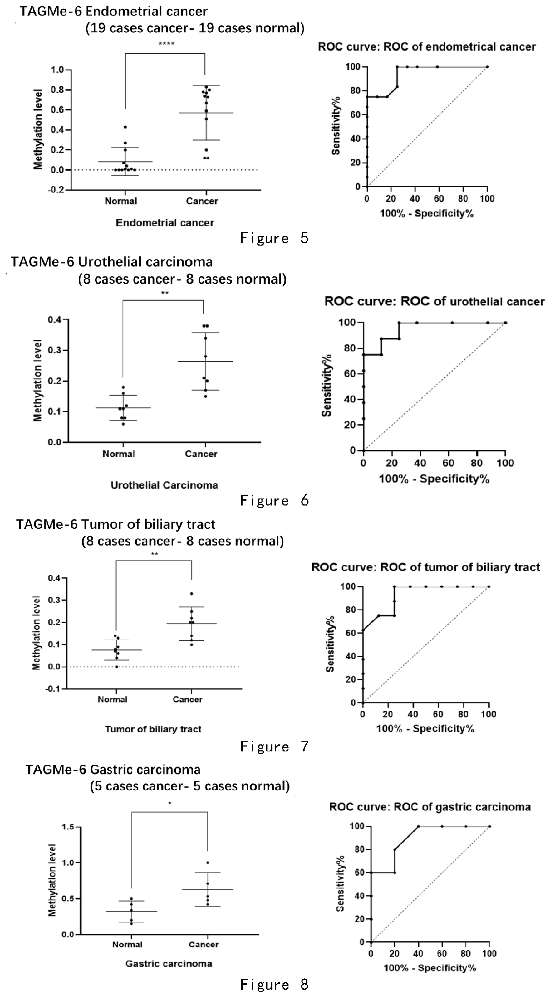

The results in Figure 5 demonstrated that in endometrial cancer clinical samples, the methylation value of TAGMe-6 in cancer tissues was significantly higher than that in paracancerous tissues (****P < 0.0001). Sensitivity was 75% and specificity was 100%.

### Example 11. TAGMe-6: Urothelial carcinoma clinical sample assay - NGS sequencing

8 paracancerous samples of urothelial carcinoma were obtained and used as the control group. 8 urothelial carcinoma samples were obtained and used as the experimental group. According to the primer combination described in Example 4 above, two rounds of PCR were performed to construct an NGS library of urothelial carcinoma clinical samples. The methylation level of TAGMe-6 was analyzed following the NGS sequencing procedure.

The results in Figure 6 demonstrated that in urothelial carcinoma clinical samples, the methylation value of TAGMe-6 in cancer tissues was significantly higher than that in paracancerous tissues (**P < 0.01). Sensitivity was 87.5% and specificity was 87.5%.

### Example 12. TAGMe-6: Biliary tract tumor clinical sample assay - NGS sequencing

8 paracancerous samples of biliary tract tumor were obtained and used as the control group. 8 biliary tract tumor samples were obtained and used as the experimental group. According to the primer combination described in Example 4 above, two rounds of PCR were performed to construct an NGS library of biliary tract tumor clinical samples. The methylation level of TAGMe-6 was analyzed following the NGS sequencing procedure.

The results Figure 7 demonstrated that in clinical samples of biliary tract tumor, the methylation value of TAGMe-6 in cancer tissues was significantly higher than that in paracancerous tissues (**P < 0.01). Sensitivity was 100% and specificity was 75%.

### Example 13. TAGMe-6: Gastric carcinoma clinical sample assay - NGS sequencing

5 paracancerous samples of gastric carcinoma were obtained and used as the control group. 5 gastric carcinoma samples were obtained and used as the experimental group. According to the primer combination described in Example 4 above, two rounds of PCR were performed to construct an NGS library of gastric carcinoma clinical samples. The methylation level of TAGMe-6 was analyzed following the NGS sequencing procedure.

The results in Figure 8 demonstrated that in gastric carcinoma clinical samples, the methylation value of TAGMe-6 in cancer tissues was significantly higher than that in paracancerous tissues (*P < 0.05). Sensitivity was 80% and specificity was 80%.

### Example 14. TAGMe-6: Breast cancer clinical sample assay - NGS sequencing

5 paracancerous samples of breast cancer were obtained and used as the control group. 5 breast cancer samples were obtained and used as the experimental group. According to the primer combination described in Example 4 above, two rounds of PCR were performed to construct an NGS library of breast cancer clinical samples. The methylation level of TAGMe-6 was analyzed following the NGS sequencing procedure.

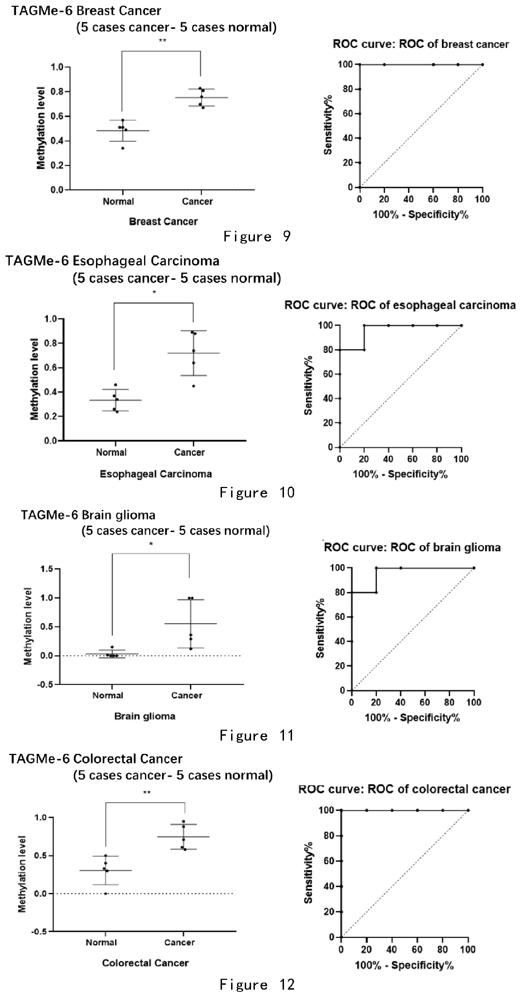

The results in Figure 9 demonstrated that in breast cancer clinical samples, the methylation value of TAGMe-6 in cancer tissues was significantly higher than that in paracancerous tissues (**P < 0.01). Sensitivity was 100% and specificity was 100%.

### Example 15. TAGMe-6: Esophageal carcinoma clinical sample assay - NGS sequencing

5 paracancerous samples of esophageal carcinoma were obtained and used as the control group. 5 esophageal carcinoma samples were obtained and used as the experimental group. According to the primer combination described in Example 4 above, two rounds of PCR were performed to construct an NGS library of esophageal carcinoma clinical samples. The methylation level of TAGMe-6 was analyzed following the NGS sequencing procedure.

The results in Figure 10 demonstrated that in clinical samples of esophageal carcinoma, the methylation value of TAGMe-6 in cancer tissues was significantly higher than that in paracancerous tissues (*P < 0.05). Sensitivity was 100% and specificity was 80%.

### Example 16. TAGMe-6: Brain glioma clinical sample assay - NGS sequencing

5 paracancerous samples of brain glioma were obtained and used as the control group. 5 brain glioma samples were obtained and used as the experimental group. According to the primer combination described in Example 4 above, two rounds of PCR were performed to construct an NGS library of brain glioma clinical samples. The methylation level of TAGMe-6 was analyzed following the NGS sequencing procedure.

The results in Figure 11 demonstrated that in brain glioma clinical samples, the methylation value of TAGMe-6 in cancer tissues was significantly higher than that in paracancerous tissues (*P < 0.05). Sensitivity was 80% and specificity was 100%.

### Example 17. TAGMe-6: Colorectal cancer clinical sample assay - NGS sequencing

5 paracancerous samples of colorectal cancer were obtained and used as the control group. 5 colorectal cancer samples were obtained and used as the experimental group. According to the primer combination described in Example 4 above, two rounds of PCR were performed to construct an NGS library of colorectal cancer clinical samples. The methylation level of TAGMe-6 was analyzed following the NGS sequencing procedure.

The results in Figure 12 demonstrated that in colorectal cancer clinical samples, the methylation value of TAGMe-6 in cancer tissues was significantly higher than that in paracancerous tissues (**P < 0.01). Sensitivity was 100% and specificity was 100%.

### Example 18. TAGMe-6: Leukaemia clinical sample assay - NGS sequencing

5 paracancerous samples of leukaemia were obtained and used as the control group. 5 leukaemia samples were obtained and used as the experimental group. According to the primer combination described in Example 4 above, two rounds of PCR were performed to construct an NGS library of leukaemia clinical samples. The methylation level of TAGMe-6 was analyzed following the NGS sequencing procedure.

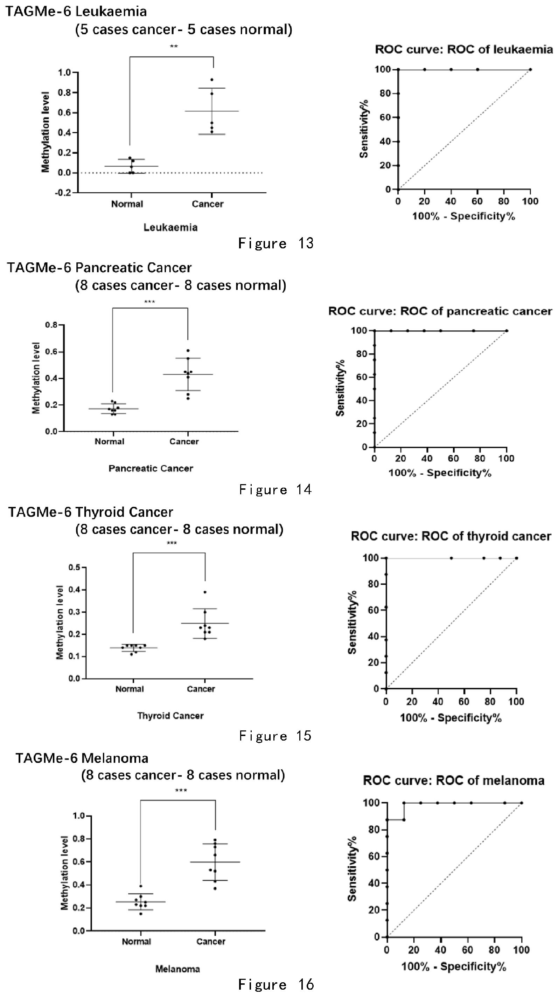

The results are shown in Figure 13, where it is demonstrated that in leukaemia clinical samples, the methylation value of TAGMe-6 in leukaemia bone marrow smears was significantly higher than that in non-leukaemia bone marrow smears (**P < 0.01). Sensitivity was 100% and specificity was 100%.

### Example 19. TAGMe-6: Pancreatic cancer clinical sample assay - NGS sequencing

8 paracancerous samples of pancreatic cancer were obtained and used as the control group. 8 pancreatic cancer samples were obtained and used as the experimental group. According to the primer combination described in Example 4 above, two rounds of PCR were performed to construct an NGS library of pancreatic cancer clinical samples. The methylation level of TAGMe-6 was analyzed following the NGS sequencing procedure.

The results in Figure 14 demonstrated that in pancreatic cancer clinical samples, the methylation value of TAGMe-6 in cancer tissues was significantly higher than that in paracancerous tissues (***P < 0.001). Sensitivity was 100% and specificity was 100%.

### Example 20. TAGMe-6: Thyroid cancer clinical sample assay - NGS sequencing

8 paracancerous samples of thyroid cancer were obtained and used as the control group. 8 thyroid cancer samples were obtained and used as the experimental group. According to the primer combination described in Example 4 above, two rounds of PCR were performed to construct an NGS library of thyroid cancer clinical samples. The methylation level of TAGMe-6 was analyzed following the NGS sequencing procedure.

The results in Figure 15 demonstrated that in thyroid cancer clinical samples, the methylation value of TAGMe-6 in cancer tissues was significantly higher than that in paracancerous tissues (***P < 0.001). Sensitivity was 100% and specificity was 100%.

### Example 21. TAGMe-6: Melanoma clinical sample assay - NGS sequencing

8 paracancerous samples of melanoma were obtained and used as the control group. 8 melanoma samples were obtained and used as the experimental group. According to the primer combination described in Example 4 above, two rounds of PCR were performed to construct an NGS library of melanoma clinical samples. The methylation level of TAGMe-6 was analyzed following the NGS sequencing procedure.

The results in Figure 16 demonstrated that in melanoma clinical samples, the methylation value of TAGMe-6 in cancer tissues was significantly higher than that in normal tissues (***P < 0.001). Sensitivity was 100% and specificity was 87.5%.

### Example 22. TAGMe-6: Nasopharyngeal carcinoma clinical sample assay - NGS sequencing

8 paracancerous samples of nasopharyngeal carcinoma were obtained and used as the control group. 8 nasopharyngeal carcinoma samples were obtained and used as the experimental group. According to the primer combination described in Example 4 above, two rounds of PCR were performed to construct an NGS library of nasopharyngeal carcinoma clinical samples. The methylation level of TAGMe-6 was analyzed following the NGS sequencing procedure.

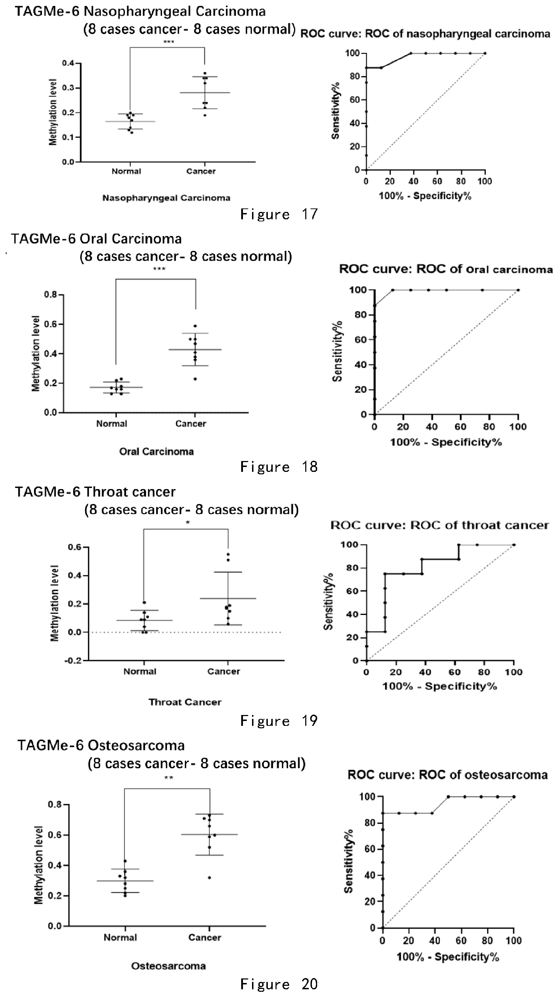

The results in Figure 17 demonstrated that in nasopharyngeal carcinoma clinical samples, the methylation value of TAGMe-6 in cancer tissues was significantly higher than that in paracancerous tissues (***P < 0.001). Sensitivity was 87.5% and specificity was 100%.

### Example 23. TAGMe-6: Oral carcinoma clinical sample assay - NGS sequencing

8 paracancerous samples of oral carcinoma were obtained and used as the control group. 8 oral carcinoma samples were obtained and used as the experimental group. According to the primer combination described in Example 4 above, two rounds of PCR were performed to construct an NGS library of oral carcinoma clinical samples. The methylation level of TAGMe-6 was analyzed following the NGS sequencing procedure.

The results in Figure 18 demonstrated that in oral carcinoma clinical samples, the methylation value of TAGMe-6 in cancer tissues was significantly higher than that in paracancerous tissues (***P < 0.001). Sensitivity was 100% and specificity was 87.5%.

### Example 24. TAGMe-6: Throat cancer clinical sample assay - NGS sequencing

8 paracancerous samples of throat cancer were obtained and used as the control group. 8 throat cancer samples were obtained and used as the experimental group. According to the primer combination described in Example 4 above, two rounds of PCR were performed to construct an NGS library of throat cancer clinical samples. The methylation level of TAGMe-6 was analyzed following the NGS sequencing procedure.

The results in Figure 19 demonstrated that in throat cancer clinical samples, the methylation value of TAGMe-6 in cancer tissues was significantly higher than that in paracancerous tissues (*P < 0.05). Sensitivity was 75% and specificity was 87.5%.

### Example 25. TAGMe-6: Osteosarcoma clinical sample assay - NGS sequencing

8 paracancerous samples of osteosarcoma were obtained and used as the control group. 8 osteosarcoma samples were obtained and used as the experimental group. According to the primer combination described in Example 4 above, two rounds of PCR were performed to construct an NGS library of osteosarcoma clinical samples. The methylation level of TAGMe-6 was analyzed following the NGS sequencing procedure.

The results in Figure 20 demonstrated that in osteosarcoma clinical samples, the methylation value of TAGMe-6 in cancer tissues was significantly higher than that in paracancerous tissues (**P < 0.01). Sensitivity was 87.5% and specificity was 87.5%.

### Example 26. TAGMe-6: Lymphoma clinical sample assay - NGS sequencing

8 paracancerous samples of lymphoma were obtained and used as the control group. 8 lymphoma samples were obtained and used as the experimental group. According to the primer combination described in Example 4 above, two rounds of PCR were performed to construct an NGS library of lymphoma clinical samples. The methylation level of TAGMe-6 was analyzed following the NGS sequencing procedure.

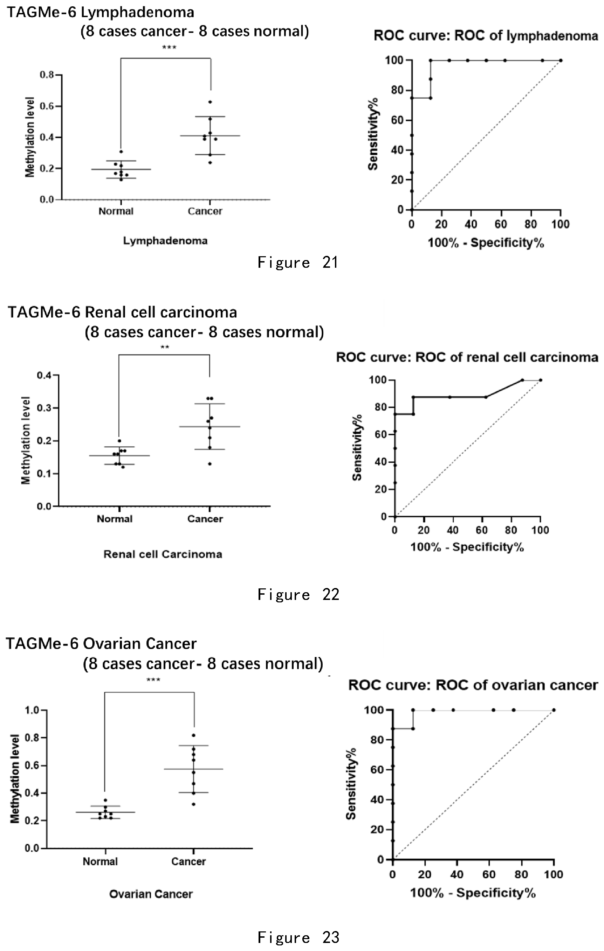

The results in Figure 21 demonstrated that in clinical samples of lymphoma, the methylation value of TAGMe-6 in cancer tissues was significantly higher than that in normal lymphoid tissues (***P < 0.001). Sensitivity was 100% and specificity was 87.5%.

### Example 27. TAGMe-6: Renal cell carcinoma clinical sample assay - NGS sequencing

8 paracancerous samples of renal cell carcinoma were obtained and used as the control group. 8 renal cell carcinoma samples were obtained and used as the experimental group. According to the primer combination described in Example 4 above, two rounds of PCR were performed to construct an NGS library of renal cell carcinoma clinical samples. The methylation level of TAGMe-6 was analyzed following the NGS sequencing procedure.

The results in Figure 22 demonstrated that in clinical samples of renal cell carcinoma, the methylation value of TAGMe-6 in cancer tissues was significantly higher than that in paracancerous tissues (**P < 0.01). Sensitivity was 87.5% and specificity was 87.5%.

### Example 28. TAGMe-6: Ovarian cancer clinical sample assay - NGS sequencing

8 paracancerous samples of ovarian cancer were obtained and used as the control group. 8 ovarian cancer samples were obtained and used as the experimental group. According to the primer combination described in Example 4 above, two rounds of PCR were performed to construct an NGS library of ovarian cancer clinical samples. The methylation level of TAGMe-6 was analyzed following the NGS sequencing procedure.

The results in Figure 23 demonstrated that in ovarian cancer clinical samples, the methylation value of TAGMe-6 in cancer tissues was significantly higher than that in paracancerous tissues (***P < 0.001). Sensitivity was 100% and specificity was 87.5%.

### Example 29. Methylation differences of TAGMe-6 CpG sites in tumor cells and non-tumor cells-Bisulfite Sequencing PCR (BSP)

The procedure for bisulfite sequencing PCR was as follows:
1. Genomic DNA was extracted from hematological tumor cell lines (myeloid leukemia cell line K562), colorectal cancer cell line (HCT116), pancreatic cancer cell line (SW1990), human renal clear cell adenocarcinoma cell line (786-O), gastric cancer cell line (BGC-823), breast cancer cell line (BT-549), and cervical cancer cell line (HeLa), as well as from their corresponding normal cells.
2. The extracted genomic DNA from cancer cell lines and normal cell lines was treated with bisulfite and used as templates for subsequent PCR amplification.
3. Amplification primers were designed based on the sequence of SEQ ID NO: 2, as shown in Table 5, and PCR amplification was performed.
4. After PCR amplification, the specificity of the PCR fragments was assessed by 2% agarose gel electrophoresis. The target fragments were recovered by gel extraction, ligated into a T-vector, and transformed into competent Escherichia coli. The bacteria were spread on plates, and colonies were selected the next day for sequencing. More than ten clones were selected for each fragment and subjected to Sanger sequencing.

**Table 5. BSP primers**

| Primer Name | 5'-3' Primer Sequence | detected CpG site No. |
|---|---|---|
| F3 | GGTTTTTGTAGTTTTYGTYGAAGAT (SEQ ID NO: 27) | CpG sites Nos. 1-54 |
| R3 | ACCTAACTACTTAAAAACAATCAAC (SEQ ID NO: 28) | |
| F4 | GTTAGGATTTTTATTAGGTTTTGTT (SEQ ID NO: 29) | CpG sites Nos. 69-152 |
| R4 | CCTAACAAACTAATCRACAACTCTA (SEQ ID NO: 30) | |

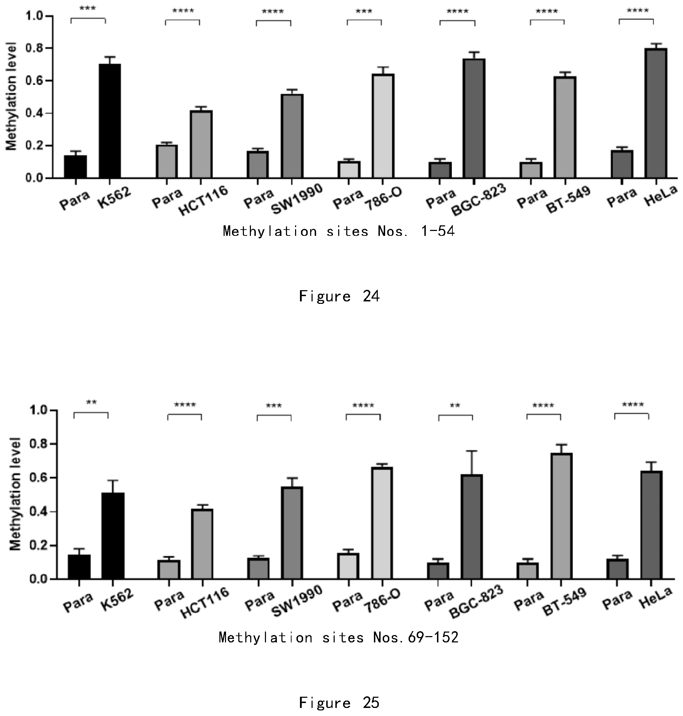

BSP assay for methylation levels at methylation sites Nos. 1 to 54 in the region of SEQ ID NO: 1 in cancer cells and normal control cells is shown in Figure 24. The results demonstrated that the methylation level of TAGMe-6 in cancer cells was significantly higher than that in normal cells.

BSP assay for methylation levels at methylation sites Nos. 69 to 152 in the region of SEQ ID NO: 1 in cancer cells and normal control cells is shown in Figure 25. The results demonstrated that the methylation level of TAGMe-6 in cancer cells was significantly higher than that in normal cells.

### Example 30. Analysis of individual CpG sites for detection

Using the clinical samples obtained in the preceding examples, the feasibility of using each of CpG sites Nos. 55 to 68 (CpG sites within the region of positions 619 to 703 in SEQ ID NO: 1) as a single CpG site for cancer detection was analyzed. The methylation status of individual CpG sites was determined by NGS sequencing.

The results in Tables 6 to 10 demonstrated that individual CpG sites already exhibited high sensitivity and specificity, serving as targets for methylation analysis, and it is significant for cancer diagnosis.

**Table 6**

| CG site | Lung Cancer | | Liver Cancer | | Prostate Cancer | | Cervical Cancer | | Endometrial Cancer | |
|---|---|---|---|---|---|---|---|---|---|---|
| | Sensitivity | Specificity | Sensitivity | Specificity | Sensitivity | Specificity | Sensitivity | Specificity | Sensitivity | Specificity |
| 55 | 95 | 95 | 80 | 75 | 78.57 | 85.71 | 84.21 | 89.47 | 84.21 | 78.95 |
| 56 | 95 | 90 | 85 | 75 | 85.71 | 78.57 | 84.21 | 84.21 | 89.47 | 73.68 |
| 57 | 85 | 85 | 70 | 65 | 78.57 | 78.57 | 94.74 | 84.21 | 84.21 | 94.74 |
| 58 | 95 | 85 | 80 | 65 | 71.43 | 78.57 | 89.47 | 73.68 | 78.95 | 84.21 |
| 59 | 90 | 90 | 80 | 65 | 71.43 | 85.71 | 94.74 | 73.68 | 89.47 | 73.68 |
| 60 | 90 | 90 | 80 | 80 | 57.14 | 78.57 | 89.47 | 78.95 | 84.21 | 84.21 |
| 61 | 95 | 95 | 65 | 75 | 78.57 | 85.71 | 84.21 | 78.95 | 89.47 | 94.74 |
| 62 | 95 | 95 | 75 | 70 | 64.29 | 64.29 | 73.68 | 89.47 | 89.47 | 89.47 |
| 63 | 90 | 95 | 65 | 70 | 92.86 | 78.57 | 89.47 | 84.21 | 73.68 | 89.47 |
| 64 | 95 | 95 | 70 | 75 | 85.71 | 78.57 | 78.95 | 94.74 | 94.74 | 84.21 |
| 65 | 90 | 85 | 75 | 85 | 78.57 | 85.71 | 78.95 | 94.74 | 89.47 | 89.47 |
| 66 | 85 | 90 | 80 | 85 | 71.43 | 85.71 | 89.47 | 89.47 | 84.21 | 78.95 |
| 67 | 95 | 95 | 70 | 85 | 85.71 | 85.71 | 84.21 | 78.95 | 89.47 | 84.21 |
| 68 | 85 | 90 | 70 | 85 | 71.43 | 85.71 | 84.21 | 84.21 | 84.21 | 78.95 |

**Table 7**

| CG site | Urothelial Carcinoma | | Biliary Tract Tumor | | Gastric Cancer | | Breast Cancer | | Esophageal Cancer | |
|---|---|---|---|---|---|---|---|---|---|---|
| | Sensitivity | Specificity | Sensitivity | Specificity | Sensitivity | Specificity | Sensitivity | Specificity | Sensitivity | Specificity |
| 55 | 87.5 | 87.5 | 75 | 87.5 | 60 | 80 | 60 | 80 | 60 | 80 |
| 56 | 75 | 62.5 | 87.5 | 75 | 80 | 60 | 80 | 60 | 60 | 80 |
| 57 | 75 | 75 | 75 | 62.5 | 60 | 80 | 80 | 100 | 80 | 80 |
| 58 | 87.5 | 75 | 75 | 75 | 80 | 80 | 80 | 80 | 80 | 80 |
| 59 | 75 | 87.5 | 87.5 | 87.5 | 80 | 80 | 80 | 80 | 60 | 60 |
| 60 | 62.5 | 87.5 | 62.5 | 87.5 | 60 | 80 | 80 | 80 | 60 | 80 |
| 61 | 87.5 | 75 | 87.5 | 75 | 100 | 60 | 60 | 60 | 60 | 80 |
| 62 | 75 | 87.5 | 75 | 87.5 | 60 | 80 | 100 | 80 | 80 | 60 |
| 63 | 62.5 | 75 | 87.5 | 75 | 80 | 80 | 80 | 60 | 60 | 80 |
| 64 | 87.5 | 75 | 75 | 62.5 | 60 | 60 | 80 | 100 | 80 | 60 |
| 65 | 75 | 87.5 | 87.5 | 75 | 80 | 60 | 80 | 80 | 60 | 80 |
| 66 | 87.5 | 87.5 | 75 | 87.5 | 80 | 80 | 60 | 80 | 80 | 80 |
| 67 | 75 | 75 | 62.5 | 75 | 60 | 60 | 80 | 60 | 80 | 60 |
| 68 | 87.5 | 62.5 | 87.5 | 62.5 | 80 | 80 | 60 | 80 | 80 | 80 |

**Table 8**

| CG site | Leukaemia | | Pancreatic Cancer | | Thyroid Cancer | | Melanoma | | Nasopharyngeal Carcinoma | |
|---|---|---|---|---|---|---|---|---|---|---|
| | Sensitivity | Specificity | Sensitivity | Specificity | Sensitivity | Specificity | Sensitivity | Specificity | Sensitivity | Specificity |
| 55 | 80 | 60 | 62.5 | 62.5 | 87.5 | 62.5 | 62.5 | 75 | 62.5 | 75 |
| 56 | 60 | 80 | 87.5 | 75 | 87.5 | 87.5 | 87.5 | 75 | 62.5 | 62.5 |
| 57 | 80 | 60 | 75 | 62.5 | 62.5 | 62.5 | 87.5 | 62.5 | 62.5 | 75 |
| 58 | 60 | 80 | 62.5 | 75 | 75 | 62.5 | 75 | 87.5 | 75 | 62.5 |
| 59 | 60 | 60 | 62.5 | 87.5 | 62.5 | 87.5 | 87.5 | 87.5 | 62.5 | 75 |
| 60 | 60 | 80 | 75 | 87.5 | 62.5 | 75 | 87.5 | 62.5 | 87.5 | 75 |
| 61 | 80 | 60 | 75 | 62.5 | 75 | 62.5 | 62.5 | 62.5 | 87.5 | 87.5 |
| 62 | 60 | 80 | 87.5 | 87.5 | 75 | 75 | 87.5 | 62.5 | 62.5 | 87.5 |
| 63 | 80 | 80 | 62.5 | 62.5 | 87.5 | 62.5 | 87.5 | 75 | 87.5 | 75 |
| 64 | 80 | 60 | 62.5 | 62.5 | 87.5 | 75 | 87.5 | 75 | 75 | 87.5 |
| 65 | 80 | 80 | 87.5 | 62.5 | 87.5 | 87.5 | 87.5 | 87.5 | 75 | 62.5 |
| 66 | 60 | 60 | 62.5 | 87.5 | 62.5 | 75 | 62.5 | 87.5 | 75 | 75 |
| 67 | 80 | 60 | 87.5 | 62.5 | 75 | 62.5 | 62.5 | 87.5 | 62.5 | 75 |
| 68 | 80 | 60 | 87.5 | 87.5 | 62.5 | 62.5 | 62.5 | 87.5 | 62.5 | 75 |

**Table 9**

| CG site | Oral Carcinoma | | Throat Cancer | | Osteosarcoma | | Lymphoma | | Renal Cell Carcinoma | |
|---|---|---|---|---|---|---|---|---|---|---|
| | Sensitivity | Specificity | Sensitivity | Specificity | Sensitivity | Specificity | Sensitivity | Specificity | Sensitivity | Specificity |
| 55 | 62.5 | 87.5 | 75 | 62.5 | 75 | 75 | 75 | 75 | 87.5 | 62.5 |
| 56 | 75 | 62.5 | 87.5 | 87.5 | 62.5 | 75 | 75 | 87.5 | 62.5 | 75 |
| 57 | 75 | 62.5 | 87.5 | 75 | 75 | 62.5 | 87.5 | 62.5 | 87.5 | 75 |
| 58 | 75 | 75 | 75 | 62.5 | 75 | 75 | 75 | 75 | 62.5 | 62.5 |
| 59 | 62.5 | 75 | 87.5 | 62.5 | 87.5 | 75 | 87.5 | 75 | 87.5 | 75 |
| 60 | 75 | 87.5 | 87.5 | 75 | 75 | 62.5 | 62.5 | 75 | 75 | 87.5 |
| 61 | 87.5 | 62.5 | 75 | 75 | 87.5 | 62.5 | 87.5 | 62.5 | 87.5 | 75 |
| 62 | 87.5 | 87.5 | 75 | 62.5 | 62.5 | 87.5 | 75 | 75 | 62.5 | 62.5 |
| 63 | 87.5 | 75 | 75 | 75 | 62.5 | 87.5 | 75 | 75 | 87.5 | 62.5 |
| 64 | 75 | 62.5 | 75 | 75 | 87.5 | 75 | 62.5 | 87.5 | 75 | 62.5 |
| 65 | 75 | 87.5 | 87.5 | 75 | 75 | 75 | 87.5 | 75 | 75 | 87.5 |
| 66 | 87.5 | 62.5 | 75 | 75 | 62.5 | 87.5 | 62.5 | 62.5 | 62.5 | 87.5 |
| 67 | 75 | 62.5 | 87.5 | 87.5 | 87.5 | 87.5 | 87.5 | 87.5 | 87.5 | 62.5 |
| 68 | 87.5 | 87.5 | 75 | 62.5 | 75 | 62.5 | 75 | 87.5 | 75 | 62.5 |

**Table 10**

| CG site | Ovarian Cancer | | Brain Glioma | | Colorectal Cancer | |
|---|---|---|---|---|---|---|
| | Sensitivity | Specificity | Sensitivity | Specificity | Sensitivity | Specificity |
| 55 | 87.5 | 87.5 | 60 | 60 | 80 | 80 |
| 56 | 62.5 | 62.5 | 80 | 60 | 60 | 60 |
| 57 | 75 | 75 | 60 | 80 | 80 | 60 |
| 58 | 75 | 87.5 | 80 | 80 | 80 | 80 |
| 59 | 87.5 | 87.5 | 80 | 60 | 80 | 60 |
| 60 | 87.5 | 75 | 80 | 60 | 60 | 80 |
| 61 | 62.5 | 87.5 | 80 | 60 | 100 | 80 |
| 62 | 62.5 | 87.5 | 60 | 80 | 100 | 60 |
| 63 | 62.5 | 75 | 80 | 60 | 60 | 80 |
| 64 | 87.5 | 87.5 | 60 | 80 | 60 | 80 |
| 65 | 75 | 75 | 60 | 60 | 60 | 60 |
| 66 | 62.5 | 62.5 | 60 | 80 | 80 | 80 |
| 67 | 87.5 | 62.5 | 60 | 80 | 80 | 80 |
| 68 | 62.5 | 62.5 | 80 | 80 | 80 | 100 |

All documents mentioned in the present disclosure are incorporated herein by reference, as if each document was individually incorporated by reference. In addition, it should be understood that, after reading the above teachings of the present disclosure, those skilled in the art may make various changes or modifications to the present disclosure, and such equivalent forms also fall within the scope defined by the appended claims of the present disclosure.

## Claims

1. A use of an isolated polynucleotide or a polynucleotide converted therefrom in the preparation of an agent or a kit for detecting tumors, wherein the polynucleotide comprises:
(1) a polynucleotide TAGMe-6 with a nucleotide sequence as shown in SEQ ID NO: 1, or a polynucleotide fragment comprising at least one CpG site with modification thereof; or
(2) a polynucleotide complementary in sequence to the polynucleotide or fragment of (1);
wherein the polynucleotide converted from the isolated polynucleotide is a polynucleotide corresponding to (1) or (2), wherein unmodified cytosine is converted to T or U, while cytosine C of the CpG site(s) with modification is unchanged.

2. The use according to claim 1, wherein the polynucleotide converted from the isolated polynucleotide is a polynucleotide with a nucleotide sequence as shown in SEQ ID NO:2; or
the at least one CpG site with modification is any "CpG" selected from sites Nos. 1 to 152 of the polynucleotide with the nucleotide sequence as shown in SEQ ID NO: 1, or a combination thereof; preferably any CpG site selected from sites Nos. 55 to 68, sites Nos. 1 to 54, or sites Nos. 69 to 152 of the polynucleotide with the nucleotide sequence as shown in SEQ ID NO: 1, or a combination thereof; or
the polynucleotide fragment is a polynucleotide with a nucleotide sequence shown in positions 619 to 703 or positions 589 to 731 of SEQ ID NO: 1.

3. The use according to claim 1, wherein the tumors comprise: respiratory system tumors, digestive system tumors, urinary system tumors, gynecological and reproductive system tumors, hematological system tumors, nervous system tumors, head and neck tumors, skin system tumors, endocrine system tumors or skeletal system tumors;
preferably, the tumors comprise: lung cancer, liver cancer, prostate cancer, cervical cancer, endometrial cancer, urothelial carcinoma, biliary tract tumor, gastric carcinoma, breast cancer, esophageal carcinoma, brain glioma, colorectal cancer, leukaemia, pancreatic cancer, thyroid cancer, melanoma, nasopharyngeal carcinoma, oral carcinoma, throat cancer, osteosarcoma, lymphadenoma, renal cell carcinoma or ovarian cancer.

4. The use according to claim 1, wherein samples for identifying tumors comprise: tissue samples, body fluid samples, blood samples.

5. A method for preparing an agent for detecting tumors, comprising:
(a) providing an isolated polynucleotide or a polynucleotide converted therefrom, comprising (1) a polynucleotide TAGMe-6 with a nucleotide sequence as shown in SEQ ID NO: 1, or a polynucleotide fragment comprising at least one CpG site with modification thereof; or (2) a polynucleotide complementary in sequence to the polynucleotide or fragment of (1); wherein the polynucleotide converted from the isolated polynucleotide is a polynucleotide corresponding to (1) or (2), wherein unmodified cytosine is converted to T or U, while cytosine C of the CpG site(s) with modification is unchanged; preferably, the at least one CpG site with modification is any CpG site selected from sites Nos. 1 to 152 of the polynucleotide with the nucleotide sequence as shown in SEQ ID NO: 1, or a combination thereof; preferably any CpG site selected from sites Nos.55 to 68, sites Nos.1 to 54, or sites Nos. 69 to 152 of the polynucleotide with the nucleotide sequence as shown in SEQ ID NO: 1, or a combination thereof; preferably, the polynucleotide fragment is a polynucleotide with a nucleotide sequence shown in positions 619 to 703 or positions 589 to 731 of SEQ ID NO: 1;
(b) using the polynucleotide of (a) as a target sequence, designing a detection agent that specifically detects the modification on CPG site(s) of the target sequence.

6. An agent or a combination of agents for specifically detecting the modification on CPG site(s) of a target sequence, wherein the target sequence is: (1) a polynucleotide TAGMe-6 with a nucleotide sequence as shown in SEQ ID NO: 1, or a polynucleotide fragment comprising at least one CpG site with modification thereof; or (2) a polynucleotide complementary in sequence to the polynucleotide or fragment of (1); wherein the polynucleotide converted from the isolated polynucleotide is a polynucleotide corresponding to (1) or (2), wherein unmodified cytosine is converted to T or U, while cytosine C of the CpG site(s) with modification is unchanged; preferably, the agent or the combination of agents is for a gene sequence comprising the target sequence, preferably, the gene sequence comprises gene Panels or gene groups; preferably, the agent or the combination of agents comprises: primers for amplifying the region from positions 619 to 703 or from positions 589 to 731 of the nucleotide sequence shown in SEQ ID NO: 1.

7. Use of the agent or combination of agents according to claim 6 in the manufacture of a kit for detecting tumors; preferably, the tumors comprise: respiratory system tumors, digestive system tumors, urinary system tumors, gynecological and reproductive system tumors, hematological system tumors, nervous system tumors, head and neck tumors, skin system tumors, endocrine system tumors or skeletal system tumors; more preferably, the tumors comprise: lung cancer, liver cancer, prostate cancer, cervical cancer, endometrial cancer, urothelial carcinoma, biliary tract tumor, gastric carcinoma, breast cancer, esophageal carcinoma, brain glioma, colorectal cancer, leukaemia, pancreatic cancer, thyroid cancer, melanoma, nasopharyngeal carcinoma, oral carcinoma, throat cancer, osteosarcoma, lymphadenoma, renal cell carcinoma or ovarian cancer.

8. A kit for detecting tumors, comprising the agent or combination of agents according to claim 6.

9. A method for analyzing the methylation level of a test sample, comprising:
(i) extracting a polynucleotide from a test sample; and
(ii) analyzing the modification on CPG site(s) of a target sequence or a fragment thereof within the extracted polynucleotide, wherein the target sequence is: (1) a polynucleotide TAGMe-6 with a nucleotide sequence as shown in SEQ ID NO: 1, or a polynucleotide fragment comprising at least one CpG site with modification thereof; or (2) a polynucleotide complementary in sequence to the polynucleotide or fragment of (1); wherein the polynucleotide converted from the isolated polynucleotide is a polynucleotide corresponding to (1) or (2), wherein unmodified cytosine is converted to T or U, while cytosine C of the CpG site(s) with modification is unchanged.

10. The method according to claim 9, wherein the method for detecting the modification on CPG site(s) of a target sequence within the extracted polynucleotide comprises: pyrosequencing, bisulfite conversion sequencing, methylation-specific PCR, methylation-sensitive restriction enzyme digestion, method using methylation chip, qPCR, digital PCR, next-generation sequencing, third-generation sequencing, whole-genome methylation sequencing, DNA enrichment detection, simplified bisulfite sequencing, HPLC, MassArray, or a combination thereof; or
the method for analyzing the modification on CPG site(s) of a target sequence within the extracted polynucleotide comprises: (i) treating the extracted polynucleotide to convert unmodified cytosines therein to uracil; preferably, the modification comprises 5-methylation modification, 5-hydroxymethylation modification, 5-formylcytosine modification or 5-carboxylcytosine modification; preferably, treating the nucleic acid of step (i) with bisulfite; and (ii) analyzing the modification of the target sequence in the nucleic acid treated by (i).

11. An isolated polynucleotide or a polynucleotide converted therefrom, wherein the polynucleotide comprises:
(1) a polynucleotide TAGMe-6 with a nucleotide sequence as shown in SEQ ID NO: 1, or a polynucleotide fragment comprising at least one CpG site with modification thereof; preferably, the at least one CpG site with modification is any CpG site selected from sites Nos. 55 to 68, sites Nos. 1 to 54, or sites Nos. 69 to 152 of the polynucleotide with the nucleotide sequence as shown in SEQ ID NO: 1, or a combination thereof; preferably the polynucleotide fragment is a nucleotide with the nucleotide sequence shown in positions 619 to 703 or positions 589 to 731 of SEQ ID NO: 1; or
(2) a polynucleotide complementary in sequence to the polynucleotide or fragment of (1);
wherein the polynucleotide converted from the isolated polynucleotide is a polynucleotide corresponding to (1) or (2), wherein unmodified cytosine is converted to T or U, while cytosine C of the CpG site(s) with modification is unchanged.
